(19) Europäisches Patentamt
European Patent Office
Office européen des brevets

(11) Veröffentlichungsnummer: **0 063 740**
**B1**

# (12) EUROPÄISCHE PATENTSCHRIFT

(45) Veröffentlichungstag der Patentschrift: **15.02.84**

(51) Int. Cl.³: **C 07 C 120/04,** C 07 C 121/16, C 07 C 121/46 // C07F7/10

(21) Anmeldenummer: **82103112.7**

(22) Anmeldetag: **13.04.82**

(54) **Verfahren zur Herstellung von tertiären Alkylcyaniden.**

(30) Priorität: **22.04.81 DE 3115976**

(43) Veröffentlichungstag der Anmeldung:
**03.11.82 Patentblatt 82/44**

(45) Bekanntmachung des Hinweises auf die Patenterteilung:
**15.02.84 Patentblatt 84/7**

(84) Benannte Vertragsstaaten:
**BE CH DE FR GB LI NL**

(56) Entgegenhaltungen:
**Chemical Abstracts Band 96, Nr. 7 15. Februar 1982 *Columbus, Ohio, USA M.T. REETZ et al. "Cyanation of* tertiary alkyl chlorides: a new method for geminal dialkylation of ketones" Seite 580, Spalte 2, Abstract Nr. 51862x**

(73) Patentinhaber: **BAYER AG, Zentralbereich Patente, Marken und Lizenzen, D-5090 Leverkusen 1, Bayerwerk (DE)**

(72) Erfinder: **Reetz, Manfred, Prof.Dr., Wiesentalweg 14, D-3550 Marburg (DE)**
Erfinder: **Chatziiosifidis, Ioannis, Dr., Ernst-Lemmer-Strasse 14/63, D-3550 Marburg (DE)**

**EP 0 063 740 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von tertiären Alkylcyaniden

Die Erfindung betrifft ein neues Verfahren zur Herstellung von tertiären Alkylcyaniden aus den entsprechenden tertiären Alkylhalogeniden. Die Verfahrensprodukte können als Zwischenprodukte, z.B. zur Herstellung von bekannten Herbiziden, verwendet werden.

Bisher ist u.W. kein generelles Verfahren zur Herstellung von tertiären Alkylcyaniden aus den entsprechenden tertiären Alkylhalogeniden durch Austausch des Halogens gegen die Nitrilgruppe bekanntgeworden. Während primäre und sekundäre Alkylhalogenide durch Kondensation mit Alkalicyaniden in die entsprechenden Nitrile überführt werden können, gelingt die analoge Umsetzung mit tertiären Alkylhalogeniden nicht. So lässt sich beispielsweise Amylchlorid mit Natriumcyanid in einer Maximalausbeute von 72% nach 96 Stunden Reaktionszeit in Capronsäurenitril überführen; sekundäres Amylchlorid oder -bromid ergeben dagegen nur 30% Nitril und tertiäres Amylchlorid oder -bromid ergeben nach diesem Verfahren überhaupt kein Nitril (vgl. Houben-Weyl, Methoden der Organischen Chemie, 4. Aufl., Bd. VIII, S. 293 [1952]). Bei der Umsetzung von Alkylhalogeniden mit Schwermetallcyaniden, wie Silber- oder Kupfercyanid, ist bekanntermassen die Bildung der entsprechenden Isonitrile begünstigt (vgl. z.B. Chem. Reviews 42, S. 189–283 (1948)).

Es wurde nun überraschend gefunden, dass man tertiäre Alkylcyanide der allgemeinen Formel

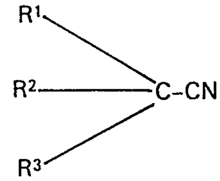

(I)

worin

$R^1$, $R^2$ und $R^3$ für gleiche oder verschiedene Alkylreste stehen oder zusammen mit dem tertiären C-Atom, an das sie gebunden sind, eine Polycycloalkylgruppe bilden, oder

$R^1$ und $R^2$ zusammen mit dem tertiären C-Atom, an das sie gebunden sind, eine Cycloalkyl- oder Bicycloalkylgruppe bilden,

in guten Ausbeuten erhält, wenn man tertiäre Alkylhalogenide der allgemeinen Formel

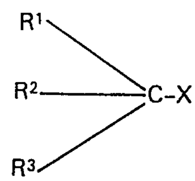

(II)

worin

X für Chlor, Brom oder Jod steht und

$R^1$, $R^2$ und $R^3$ die oben angegebene Bedeutung haben,

mit Trimethylsilylcyanid, $(CH_3)_3SiCN$ (III), in Gegenwart einer Lewis-Säure aus der Gruppe $SnCl_4$, $TiCl_4$ und $BiCl_3$ und gegebenenfalls in Anwesenheit eines Verdünnungsmittels bei Temperaturen zwischen 10 und 40°C umsetzt.

Verwendet man tert.-Butylchlorid als Ausgangsstoff, Zinn-tetrachlorid als Lewis-Säure und Dichlormethan als Verdünnungsmittel, so lässt sich der Reaktionsablauf durch das folgende Formelschema wiedergeben:

$$(CH_3)_3C\,Cl + (CH_3)_3SiCN \xrightarrow[CH_2Cl_2]{[SnCl_4]} (CH_3)_3\,CCN + (CH_3)_3SiCl$$

Die als Ausgangsstoffe zu verwendenden tertiären Alkylhalogenide sind durch die Formel (II) allgemein definiert. In dieser Formel stehen $R^1$, $R^2$ und $R^3$ vorzugsweise für Alkylreste mit 1–10 C-Atomen oder zusammen mit dem tertiären C-Atom, an das sie gebunden sind, für Polycycloalkylgruppen mit bis zu 20 C-Atomen; darüber hinaus stehen $R^1$ und $R^2$ zusammen mit dem C-Atom, an das sie gebunden sind, für Cycloalkylgruppen mit vorzugsweise 3–12 C-Atomen sowie für Bicycloalkylgruppen mit vorzugsweise 6–10 C-Atomen. Die genannten Reste können substituiert sein durch solche Substituenten, die die verwendete Lewis-Säure nicht desaktivieren. Als Beispiele hierfür seien genannt: Cyano, Fluor sowie Chlor, welches an ein primäres C-Atom gebunden ist.

X steht in Formel (II) bevorzugt für Chlor.

Unter dem Ausdruck «Alkylhalogenide» sind im Rahmen dieser Erfindung demnach ausser den offenkettigen Verbindungen auch gesättigte cycloaliphatische Halogenide zu verstehen sowie

bestimmte Substitutionsprodukte beider Grundtypen. Zu den gesättigten cycloaliphatischen Halogeniden gehören auch polycyclische Alkylhalogenide mit dem Halogenatom am Brückenkopf, z.B. die 1-Halogen-adamantane.

Die erfindungsgemäss verwendbaren tertiären Alkylhalogenide (II) sind bekannt oder können nach bekannten Verfahren hergestellt werden (vgl. z.B. Houben-Weyl, Methoden der Organischen Chemie, 4. Aufl., Bd. V/3, S. 503 ff (1960) und Bd. V/4, S. 13 ff [1962]). Als Beispiele seien genannt: t-Butylchlorid, t-Amylchlorid, 1-Chlor-1-methyl-cyclooctan, 1-Chlor-1-propylcyclohexan, 2-Chlor-2-methyl-[2.2.2]-bicyclooctan, 1-Brom-adamantan. – Besonders bevorzugt ist t-Butylchlorid.

Trimethylsilylcyanid (III) ist bekannt (vgl. z.B. Synthesis 1979, S. 522 und 523, ferner DE-OS 30 18 821).

Die erfindungsgemässe Umsetzung wird bevorzugt in Anwesenheit eines Verdünnungsmittels als inertes Solvens durchgeführt. Hierfür besonders geeignet sind bestimmte chlorierte Kohlenwasserstoffe (die selbstverständlich kein tertiär gebundenes Halogen enthalten dürfen), wie insbesondere Dichlormethan und 1,2-Dichlorethan.

Wenn ohne Solvens gearbeitet wird, werden im allgemeinen etwas geringere Ausbeuten erhalten.

Die erfindungsgemässe Umsetzung wird in Gegenwart einer Lewis-Säure durchgeführt. Geeignete Lewis-Säuren im Rahmen dieser Erfindung sind $SnCl_4$, $TiCl_4$ und $BiCl_3$; besonders bevorzugt wird $SnCl_4$ eingesetzt, da hiermit die höchsten Ausbeuten erzielt werden.

Die Reaktionstemperaturen liegen im allgemeinen zwischen etwa 10 und 40°C, vorzugsweise zwischen 15 und 25°C; am zweckmässigsten wird bei Raumtemperatur gearbeitet. Im allgemeinen wird das Verfahren bei Normaldruck durchgeführt.

Die Reaktionszeiten betragen normalerweise etwa 24–38 Stunden.

Bei der Durchführung des erfindungsgemässen Verfahrens setzt man auf 1 Mol tert.-Alkylhalogenid (II) im allgemeinen 1–1,8 Mol, vorzugsweise 1,2–1,6 Mol Trimethylsilylcyanid (III) ein.

Die Lewis-Säure wird in unterstöchiometrischer Menge, im allgemeinen in einer Menge von 10–40 Mol-%, vorzugsweise von 20–30 Mol-%, bezogen auf (II), eingesetzt. Mengen von <10 Mol-% erfordern längere Reaktionszeiten, äquivalente (stöchiometrische) Mengen dagegen hemmen die Reaktion.

Man arbeitet unter Feuchtigkeitsausschluss und darüber hinaus zweckmässigerweise auch unter einer Inertgasatmosphäre, am einfachsten unter Stickstoff.

Wenn man mit einem Verdünnungsmittel arbeitet, ist es zweckmässig, das tert.-Alkylhalogenid und das Trimethylsilylcyanid in trockenem Solvens vorzulegen und die Lewis-Säure unter einer Stickstoffatmosphäre zuzugeben.

Es ist aber auch möglich, zuerst das Trimethylsilylcyanid und die Lewis-Säure zusammenzubringen und erst dann das tert.-Alkylhalogenid zuzugeben. Wenn ohne Solvens gearbeitet wird, ist die letztere Reaktionsführung besser geeignet. Da das Mischen von Trimethylsilylcyanid und $SnCl_4$ leicht exotherm ist, sollte man in diesem Fall abkühlen lassen und erst dann das tert.-Alkylhalogenid zugeben.

In der Regel kann man die Reaktion $^1H$-NMR-spektroskopisch zeitlich leicht verfolgen. Z.B. verschwindet das Signal der tert.-Butylgruppe des tert.-Butylchlorids bei fortschreitender Umsetzung allmählich, während die Intensität des Signals der tert.-Butylgruppe des Pivalonitrils bei etwas höherem Feld langsam zunimmt. (Bei geringfügig höherem Feld ist in diesem Fall ein weiteres Singulett zu erkennen. Es ist nicht klar, ob dies eine komplexierte Form des Produkts ist; nach der wässrigen Aufarbeitung findet man es nicht wieder.)

Die Aufarbeitung geschieht in üblicher Weise (vgl. Beispielteil) und die tert.-Alkylcyanide (I) werden schliesslich durch Destillation oder Kristallisation in reiner Form isoliert.

Die nach dem erfindungsgemässen Verfahren herstellbaren tertiären Alkylcyanide (I) sind zum Teil bekannt; diese Nitrile sind wertvolle Zwischenprodukte, z.B. zur Herstellung von Herbiziden, Tensiden und Korrosionsschutzmitteln.

So kann beispielsweise Pivalonitril (A) durch katalytische Hydrierung in Neopentylamin (B) überführt werden, welches auf verschiedenen Wegen zu dem bekannten herbiziden Wirkstoff 1-Amino-6-ethylthio-3-neopentyl-1,3,5-triazin-2,4(1H, 3H)-dion (K) (vgl. z.B. DK-PS 136 067) umgesetzt werden kann:

$$(CH_3)_3C\text{-}CN \xrightarrow[\text{[Kat.]}]{2\,H_2} (CH)_3C\text{-}CH_2\text{-}NH_2$$

(A) (B)

Ein Verfahrensweg führt über folgende Stufen (vgl. BE-PS 682 820; Angew. Chem. 82 (1970), S. 63-67; Synthesis 1970, S. 542-543; DE-OS 2 254 200):

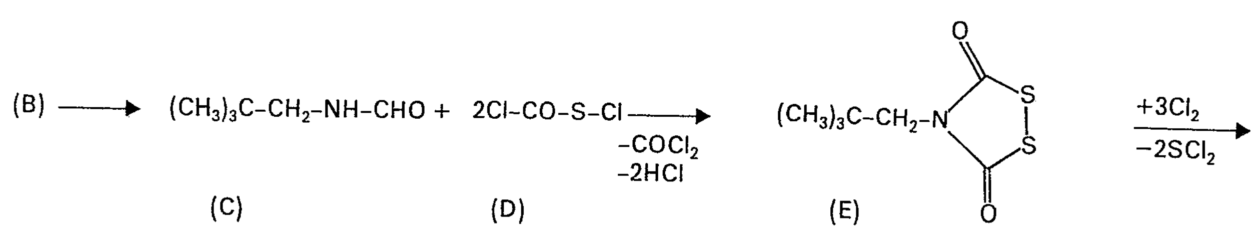


(C) (D) (E)

$(CH_3)_3C-CH_2-N(COCl)_2$ (F)

(F) +

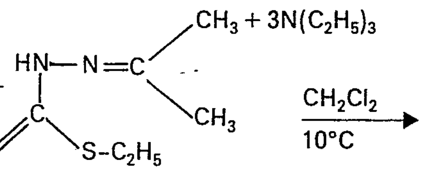


xHBr (G)

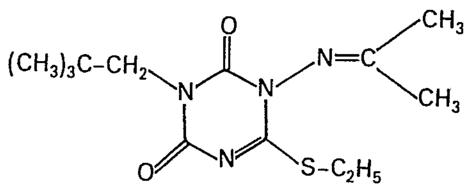

(H)

$[+2N(C_2H_5)_3 \cdot HCl + N(C_2H_5)_3 \cdot HBr]$

(H)

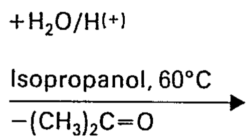


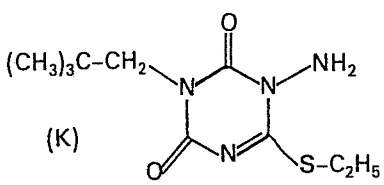

(K)

Ein anderer Verfahrensweg führt über folgende Stufen (vgl. DE-OS 3 006 226 und DE-OS 3 006 263):

(B) → $(CH_3)_3C-CH_2-N(CO-OC_6H_5)_2$

(L)

(L) + (G) → (H) → (K)

Die nachfolgenden Herstellungsbeispiele sollen zur weiteren Erläuterung der Erfindung dienen.

Beispiele

Allgemeine Vorschrift zur Herstellung von tert.-Alkylcyaniden

Zu einer Lösung von 10 mMol tert.-Alkylchlorid und 15 mMol Trimethylsilylcyanid in 30 ml trockenem Methylenchlorid werden bei Raumtemperatur unter Stickstoff langsam und unter Rühren 30 Mol-% $SnCl_4$ zugegeben. Nach 24–38 Stunden wird die Reaktionslösung (mittlererweise fast schwarz) auf Eiswasser gegossen und kräftig geschüttelt. Die organische Phase wird abgetrennt, die wässrige Phase wird zweimal mit Methylenchlorid gewaschen und die vereinigten organischen Phasen werden mit 10%iger Natriumbicarbonat-Lösung gewaschen. Die Lösung wird über Natriumsulfat getrocknet, eingeengt und destilliert.

Ausgangsprodukte (II), Verfahrensprodukte (I), Reaktionszeiten, Ausbeuten und physikalische Daten der Verfahrensprodukte gehen aus der nachfolgenden Tabelle 1 hervor:

Grössere Ansätze verlaufen in ähnlicher Weise.

Tabelle 1

| Beispiel Nr. | Ausgangsprodukt (II) | Verfahrensprodukt (I) | Reaktionszeit (h) | Ausbeuten (% d.Th.) nach NMR* | isoliert** | Siedepunkt oder Schmelzpunkt (Fp.) | Lage der $CH_3$-Gruppe von (I) im NMR-Spektrum*** |
|---|---|---|---|---|---|---|---|
| 1 | $(CH_3)_3CCl$ | $(CH_3)_3CCN$ | 36 | ~ 75 | 64 | 105–7°C | 1.3 |
| 2 | 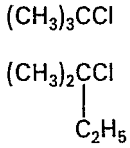 | 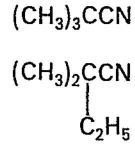 | 38 | 80 | 75 | 32°C/25 mbar | 1.3 |
| 3 | 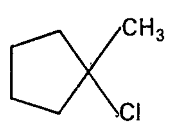 | 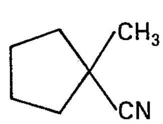 | 32 | 75 | 67 | 52°C/25 mbar | 1.3 |
| 4 | 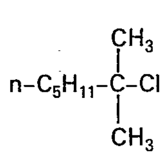 | 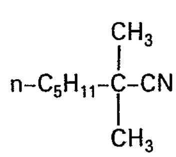 | 38 | 78 | 70 | 72°C/25 mbar | 1.3 |

Tabelle 1 (Fortsetzung)

| Bei-spiel Nr. | Ausgangs-produkt (II) | Verfahrens-produkt (I) | Reak-tions-zeit (h) | Ausbeuten (% d.Th.) nach NMR* | isoliert** | Siedepunkt oder Schmelzpunkt (Fp.) | Lage der $CH_3$-Gruppe von (I) im NMR-Spek-trum*** |
|---|---|---|---|---|---|---|---|
| 5 | 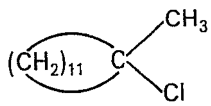 | 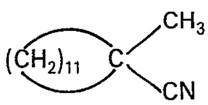 | 38 | 75 | 60 | Fp. 158–162°C | 1.3 |
| 6 | 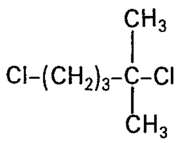 | 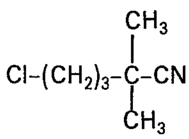 | 25 | 85 | 72 | 90°C/25 mbar | 1.3 |
| 7 | 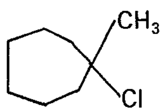 | 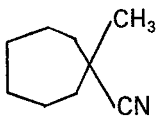 | 25 | 85 | 76 | 82°C/25 mbar | 1.3 |
| 8 | 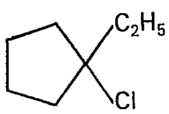 | 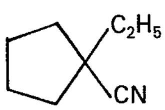 | 26 | 87 | 82 | 67°C/25 mbar | 1.1 (Triplett) |
| 9 | 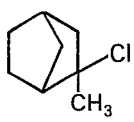 | 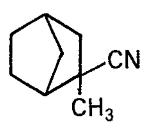 | 24 | 90 | 84 | 85°C/25 mbar | 1.4 |
| 10 | 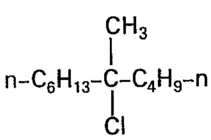 | 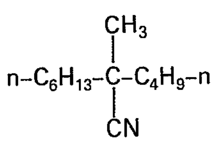 | 25 | 80 | 70 | 54°C/0,665 mbar | 1.3 |
| 11 | 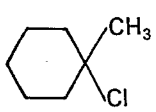 | 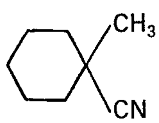 | 38 | 60 | 44 | 62°C/25 mbar | 1.3 |
| 12 | 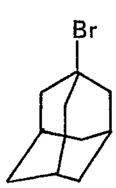 (1-Brom-adamantan) | 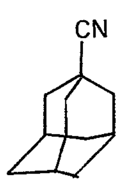 (1-Cyan-adamantan) | 30 | 38 | 29 | 194°C | – |

* gemessen durch $^1H$-NMR-spektroskopische Analyse
** Ausbeute an isoliertem Produkt
*** $^1H$-NMR-Spektrum (in $CCl_4$; Tetramethylsilan [TMS] als interner Standard)

**Patentansprüche**

1. Verfahren zur Herstellung von tertiären Alkylcyaniden der allgemeinen Formel

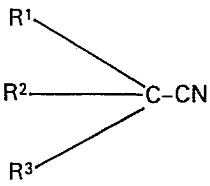

(I)

in welcher
$R^1$, $R^2$ und $R^3$ für gleiche oder verschiedene Alkylreste stehen oder zusammen mit dem tertiären C-Atom, an das sie gebunden sind, eine Polycycloalkylgruppe bilden, oder
$R^1$ und $R^2$ zusammen mit dem tertiären C-Atom, an das sie gebunden sind, eine Cycloalkyl- oder Bicycloalkylgruppe bilden,
dadurch gekennzeichnet, dass man tertiäre Alkylhalogenide der allgemeinen Formel

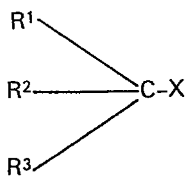

(II)

worin
X für Chlor, Brom oder Jod steht und
$R^1$, $R^2$ und $R^3$ die oben angegebene Bedeutung haben, mit Trimethylsilylcyanid, $(CH_3)_3SiCN$ (III), in Gegenwart einer Lewis-Säure aus der Gruppe $SnCl_4$, $TiCl_4$ und $BiCl_3$ und gegebenenfalls in Anwesenheit eines Verdünnungsmittels bei Temperaturen zwischen 10 und 40°C umsetzt.

2. Verfahren nach Anspruch 1, dadurch gekennzeichnet, dass man bei Temperaturen zwischen 15 und 25°C arbeitet.

3. Verfahren nach Anspruch 1, dadurch gekennzeichnet, dass man auf 1 Mol tertiäres Alkylhalogenid (II) 1-1,8 Mol, vorzugsweise 1,2-1,6 Mol, Trimethylsilylcyanid (III) einsetzt.

4. Verfahren nach Anspruch 1, dadurch gekennzeichnet, dass man auf 1 Mol tertiäres Alkylhalogenid (II) 10-40 Mol-%, vorzugsweise 20-30 Mol-% der Lewis-Säure einsetzt.

5. Verfahren nach Anspruch 1, dadurch gekennzeichnet, dass man als Lewis-Säure $SnCl_4$ einsetzt.

6. Verfahren nach Anspruch 1, dadurch gekennzeichnet, dass man in Anwesenheit eines Verdünnungsmittels arbeitet und als solches einen chlorierten Kohlenwasserstoff, der jedoch kein tertiär gebundenes Chlor enthält, verwendet.

7. Verfahren nach Anspruch 5, dadurch gekennzeichnet, dass man als Verdünnungsmittel Dichlormethan oder 1,2-Dichlormethan verwendet.

8. Verfahren nach Anspruch 1, dadurch gekennzeichnet, dass man als tertiäres Alkylhalogenid tert.-Butylchlorid einsetzt.

**Revendications**

1. Procédé pour la fabrication de cyanures d'alkyles tertiaires de formule générale

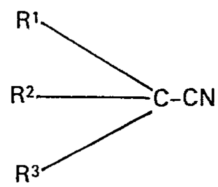

(I)

dans laquelle
$R^1$, $R^2$ et $R^3$ représentent des restes alkyles identiques ou différents ou bien, pris avec l'atome de carbone tertiaire auquel ils sont liés, forment un groupe polycycloalkyle, ou bien
$R^1$ et $R^2$ pris ensemble avec l'atome de carbone tertiaire auquel ils sont liés, forment un groupe cycloalkyle ou bicycloalkyle, caractérisé en ce que l'on fait réagir des halogénures d'alkyles tertiaires de formule générale

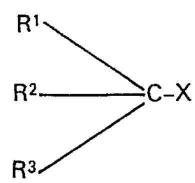

(II)

dans laquelle
X représente le chlore, le brome ou l'iode et
$R^1$, $R^2$ et $R^3$ ont les significations indiquées ci-dessus, avec le cyanure de triméthylsilyle, $(CH_3)_3SiCN$ (III), à des températures comprises entre 10 et 40°C, en présence d'un acide de Lewis choisi parmi $SnCl_4$, $TiCl_4$ et $BiCl_3$ et éventuellement en présence d'un agent diluant.

2. Procédé selon la revendication 1, caractérisé en ce que l'on opère à des températures comprises entre 15 et 25°C.

3. Procédé selon la revendication 1, caractérisé en ce que l'on utilise 1-1,8 mole de cyanure de triméthylsilyle (III), de préférence 1,2 à 1,6 mole, pour 1 mole d'halogénure d'alkyle tertiaire (II).

4. Procédé selon la revendication 1, caractérisé en ce que l'on utilise 10-40 moles % de l'acide de Lewis, de préférence 20-30 moles %, pour 1 mole d'halogénure d'alkyle tertiaire (II).

5. Procédé selon la revendication 1, caractérisé en ce que l'on utilise $SnCl_4$ comme acide de Lewis.

6. Procédé selon la revendication 1, caractérisé en ce que l'on opère en présence d'un agent diluant et on utilise comme tel un hydrocarbure chloré, mais qui ne contient pas de chlore lié à un carbone tertiaire.

7. Procédé selon la revendication 5, caractérisé en ce que l'on utilise comme agent diluant le dichlorométhane ou le 1,2-dichloroéthane.

8. Procédé selon la revendication 1, caractérisé en ce que l'on utilise comme halogénure d'alkyle tertiaire le chlorure de tert-butyle.

## Claims

1. Process for the preparation of tertiary alkyl cyanides of the general formula

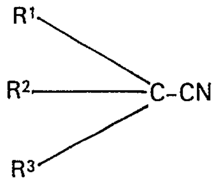

(I)

wherein

$R^1$, $R^2$ and $R^3$ represent identical or different alkyl radicals or, together with the tertiary C atom to which they are bonded, form a polycycloalkyl group, or

$R^1$ and $R^2$, together with the tertiary C atom to which they are bonded, form a cycloalkyl group or a bicycloalkyl group, characterised in that tertiary alkyl halides of the general formula

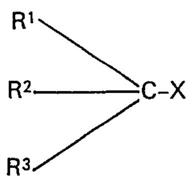

(II)

wherein

X represents chlorine, bromine or iodine and

$R^1$, $R^2$ and $R^3$ have the meaning given above,

are reacted with trimethylsilyl cyanide, $(CH_3)_3SiCN$(III), in the presence of a Lewis acid from the group comprising $SnCl_4$, $TiCl_4$ and $BiCl_3$, and, if appropriate, in the presence of a diluent, at temperatures between 10 and 40°C.

2. Process according to Claim 1, characterised in that the reaction is carried out at temperatures between 15 and 25°C.

3. Process according to Claim 1, characterised in that 1–1.8 mols, preferably 1.2–1.6 mols, of trimethylsilyl cyanide (III) are employed per mol of tertiary alkyl halide (II).

4. Process according to Claim 1, characterised in that 10–40 mol%, preferably 20–30 mol%, of the Lewis acid is employed per mol of tertiary alkyl halide (II).

5. Process according to Claim 1, characterised in that $SnCl_4$ is employed as the Lewis acid.

6. Process according to Claim 1, characterised in that the reaction is carried out in the presence of a diluent, and the diluent used is a chlorinated hydrocarbon, which, however, does not contain chlorine bonded to a tertiary carbon atom.

7. Process according to Claim 5, characterised in that dichloromethane or 1,2-dichloroethane is used as the diluent.

8. Process according to Claim 1, characterised in that tert.-butyl chloride ist employed as the tertiary alkyl halide.